# EUROPÄISCHE PATENTSCHRIFT

(11) **EP 0 468 952 B1**
(45) Veröffentlichungstag und Bekanntmachung des Hinweises auf die Patenterteilung: **26.10.1994**
(21) Anmeldenummer: 91890148.9
(22) Anmeldetag: 10.07.1991
(51) Int. Cl.: A61B 3/107, G01B 11/24

(54) **Optisches Messsystem für die menschliche Hornhaut**
Optical measuring system for human cornea
Procédé pour mesurer optiquement la cornée humaine

(30) Priorität: 24.07.1990 AT 1555/90
(43) Veröffentlichungstag der Anmeldung: 29.01.1992
(73) Patentinhaber: Spellitz, Fritz, A-1120 Wien (AT)
(72) Erfinder: Spellitz, Fritz, A-1120 Wien (AT)
(74) Vertreter: Barger, Werner, Dipl.-Ing.

(56) Entgegenhaltungen:
- EP-A- 0 210 722
- EP-A- 0 346 015
- WO-A-86/02249
- US-A- 4 093 340
- US-A- 4 925 301

## Beschreibung

Die Erfindung betrifft ein optisches Meßsystem für die menschliche Hornhaut, mit Lichtquelle, Scanner, Ablenkoptik, Sensor und Auswerteeinheit, wobei das Auge vor der Ablenkoptik positioniert ist.

Ein derartiges Meßsystem ist insbesondere aus der DE-T1-35 90 539 bekannt. Diese Druckschrift wurde zuerst in englischer Sprache als WO/86/02249 veröffentlicht. Bei der in dieser Druckschrift geoffenbarten Anordnung eines Lasermeßsystemes mit Scanner, Sensor und Auswerteeinheit wird ein holographisches Element knapp vor dem Auge als Fokusiervorrichtung verwendet. Dieses holographische Element ist so gewählt, daß im Falle einer idealen Positionierung einer idealen Cornea der von der Hornhautoberfläche reflektierte Strahl mit dem einfallenden Strahl zusammenfällt. Zur Auswertung der Daten ist es daher erforderlich, in den Strahlengang einen Strahlenteiler einzubringen, der einen Teil des reflektierten Strahles auf einen Flächensensor lenkt. Dieser Strahlenteiler lenkt gleichzeitig einen Teil des vom Laser über einen Scanner entsprechend gerichteten Strahles auf einen zweiten zweidimensionalen Sensor, dessen Ausgangssignale einen Computer davon informieren, wie der ausgehende Lichtstrahl momentan gerichtet ist. Aus diesen Daten und der Kenntnis der feststehenden oder momentanen Geometrie kann der Computer die Form der Hornhautoberfläche berechnen.

Das vorbekannte Gerät weist jedoch einige schwerwiegende Nachteile auf.

Das holographische Element muß groß bemessen sein, um immer den theoretisch richtigen Einfallswinkel auf das Auge zu bewirken. Nur dann ist es möglich, das Meßergebnis im Bereich des Flächensensors zu erhalten und so zu einem Meßwert zu kommen.

Die Flächensensoren selbst begrenzen die Meßgenauigkeit und Auflösung und sind dabei kostspielig. Um zur gewünschten Meßgenauigkeit zu kommen, ist es notwendig, entsprechend große Abstände zu wählen, wobei aber nicht nur die Strahlaufweitung an der positiv gekrümmten Hornhaut bedacht werden muß, sondern auch, daß bei Verletzungen der Hornhautoberfläche beträchtliche Abweichungen der Flächennormale vom Idealzustand auftreten können, wodurch unter Umständen mehrere Meßpunkte hintereinander keinen Meßwert liefern, da der reflektierte Strahl nicht mehr auf den Flächensensor gelangt.

Ein weiterer großer Nachteil ist im Strahlenteiler zu sehen, der zwar vorteilhafterweise die Möglichkeit bietet, den Computer mit Informationen über die momentane Lage des Strahles zu informieren, gleichzeitig aber den vom Auge reflektierten Strahl schwächt, sodaß es notwendig ist, das Auge mit einer Intensität zu bestrahlen, die hinreicht, daß der reflektierte Strahl nach der Teilreflexion am Strahlenteiler noch immer zuverlässig in der Lage ist, den Flächensensor entsprechend zu aktivieren. Diese vergrößerte Lichtintensität stellt eine äußerst unangenehme Mehrbelastung für das Auge dar.

Weitere Probleme treten dadurch auf, daß der Strahlengang vom Scanner zum Referenzflächensensor naturgemäß wesentlich kürzer ist als der Strahlengang des reflektierten Strahles, wobei der Einfalls- und somit auch der Ausfallswinkel des ausgehenden Strahles auf den Strahlteiler gleich groß ist wie die entsprechenden Winkel des reflektierten Strahles, wobei jedoch über den gesamten Meßbereich, projiziert auf den Strahlteiler gesehen, die ausgehenden Strahlen und damit auch ihr Bild am Referenzflächensensor divergieren, was zwar der Meßgenauigkeit nutzt, aber wiederum eine Einschränkung bei den Anordnungsmöglichkeiten des Scanners, Strahlteilers und Referenzflächensensors darstellt.

Ein anderes optisches Abtastsystem, allerdings zum Erfassen großer ebener Flächen, ist aus der DE-OS-25 24 152 bekannt. Bei diesem System wird ein rotierender Lichtstrahl durch feststehende Ablenkspiegel so reflektiert, daß er im Zuge seiner Rotation das gesamte abzutastende Gebiet erfaßt. Auf welche Weise die eigentliche Meßwerterfassung erfolgt (reflektierter Strahl, durchgehender Strahl, Art des Scanners, etc.), wird nicht geoffenbart.

Es ist das Ziel der Erfindung, ein Meßsystem zu entwickeln, das die Nachteile nicht aufweist, das insbesondere die Intensität des auf das Auge auftreffenden Laserstrahles so gering wie nur möglich hält, das ohne kostspieliges und sensibles holographisches Element auskommt und das es insbesondere ermöglicht, empfindlichere, genauere und trotzdem kostengünstigere Linearsensoren statt der Flächensensoren zu verwenden.

Diese Ziele werden erfindungsgemäß durch die im kennzeichnenden Teil von Anspruch 1 angeführten Merkmale erreicht.

Durch diese Maßnahme wird erreicht, daß der vom Auge reflektierte Laserstrahl unmittelbar auf den Sensor fällt. Weiters erzielt man dadurch den überraschenden Effekt, daß die von den Umlenkspiegeln aufs Auge gelenkten Strahlen jeweils unter einem solchen Winkel auf den zugehörigen Bereich der Hornhautoberfläche gelangen, daß eine bestmögliche Auswertung am Sensor möglich ist, ohne daß dessen Positionierung einander widersprechende Bedingungen erfüllen muß.

Bevorzugt ist der Sensor ein Linearsensor. Diese kostengünstige und platzsparende Variante ist möglich, da die Strahlablenkung nur in einer Ebene erfolgt.

In einer vorteilhaften Ausgestaltung ist vorgesehen, daß die Flächen der Ablenkspiegel schiefwinkelig zur Ebene des rotierenden Lichtstrahles angeordnet sind und daß die Ablenkspiegel den Lichtstrahl exakt auf Punkte richten, welche einem bestimmten Radius bzw. Durchmesser einer Idealhornhautoberfläche entsprechen. Dadurch wird beim Abtastvorgang jeweils ein Hornhautradius bzw -durch-messer ausgemessen, wenn die Messanordnung um die Augenachse gedreht wird, um Radius für Radius bzw. Durchmesser für Durchmesser der Hornhautoberfläche vermessen zu können. Durch die schiefwinkelige Anordnung liegen die vermessenen Punkte der Cornea exakt auf einem Radius bzw. Durchmesser der Idealhornhautoberfläche.

Es ist selbstverständlich auch möglich, ein anderes System anzuwenden und die Meßanordnung jeweils um einen gewissen Betrag vertikal oder horizontal zu verschieben, um zu Meßpunkten zu kommen, die zumindest im wesentlichen ein kartesisches Koordinatensystem bestimmen.

Um eine bestmögliche Korrelation zwischen momentan ausgehendem Lichtstrahl und gemessenem reflektierten Lichtstrahl zu erhalten, ist in einer Ausgestaltung des Meßsystems vorgesehen, daß zumindest zwei der Ablenkspiegel den einfallenden Strahl auf ein Überwachungselement für den Strahldurchgang lenken. In Kenntnis der Geometrie und unter der praktisch immer zutreffenden Annahme, daß während des Überstreichens der Spiegel keine ins Gewicht fallenden Schwankungen in der Winkelgeschwindigkeit des Drehspiegels auftreten, kann so für jeden Meßpunkt eine hervorragende Korrelation des einfallenden mit dem reflektierten Strahl vorgenommen werden. Durch Benutzung von mehr als zwei Spiegeln wird die Genauigkeit weiter erhöht.

In einer vorteilhaften weiteren Ausgestaltung ist vorgesehen, jeweils zwei, vorzugsweise benachbarte, Ablenkspiegel auf ein- und denselben Punkt der Idealhornhautoberfläche zu richten, um so die Redundanz der Meßwerte zu erhöhen und Augenbewegungen etc. möglichst vollständig auszuschalten.

Durch die erfindungsgemäße Anordnung ist es auch möglich geworden, den bei den vorbekannten Geräten notwendigen langen Strahlengang, insbesondere des reflektierten Lichtstrahles zu vermeiden. Dies führt dazu, die Strahlaufweitung, die unweigerlich an der positiv gekrümmten Hornhautoberfläche erfolgt, klein zu halten, was es ermöglicht, feiner unterteilte Sensoren oder Analogsensoren zu verwenden, da ein schärferes Signal empfangen wird.

Die Bezeichnung "Meßpunkt" muß unter Berücksichtigung der herrschenden Verhältnisse ausgelegt werden. Durch den rotierenden Scannerspiegel und das aufeinanderfolgende Überstreichen der Ablenkspiegel werden am Auge längliche Bereiche beleuchtet, deren Reflexionen am Sensor einander im allgemeinen örtlich überlappen. Dem Rechner wird entweder ein entsprechend gemittelter Meßwert zugeführt oder es wird ein solcher Meßwert von ihm ermittelt.

Die Erfindung wird an Hand der beiliegenden Zeichnung näher erläutert. Dabei zeigt
Fig. 1 ein Ausführungsbeispiel eines erfindungsgemäßen Meßsystems in Seitenansicht,
Fig. 2 in Draufsicht und
Fig. 3 einen Ausschnitt einer Variante.

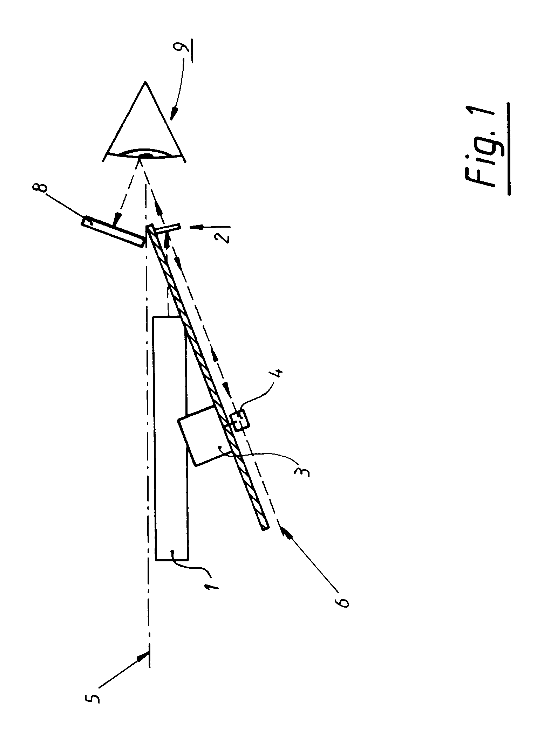

Das dargestellte Meßsystem rotiert um die optische Achse des zu vermessenden Auges, wobei die Darstellung in Abbildung 1 der Position entspricht, in der der horizontale Hornhautdurchmesser vermessen wird.

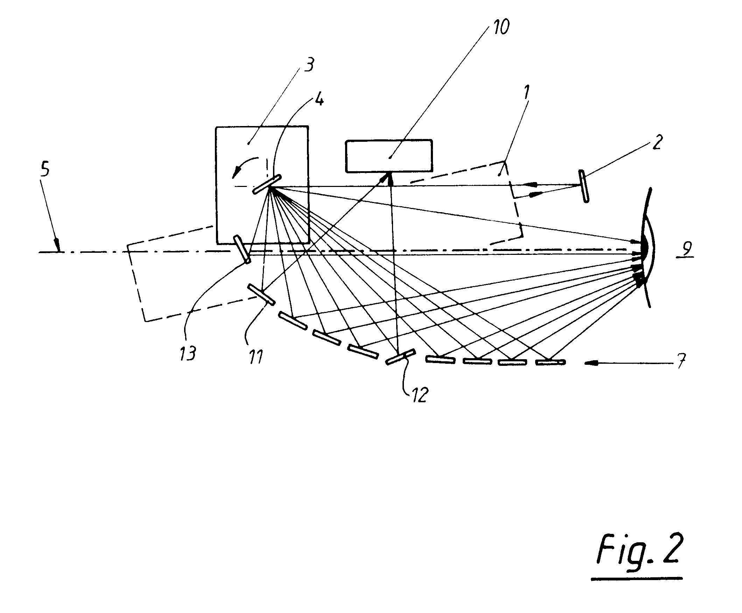

In Fig. 2 ist in der Draufsicht der Verlauf der jeweiligen über die Umlenkspiegel auf die Hornhautoberfläche gelangenden Strahlen dargestellt.

Ein Laser 1 sendet einen Lichtstrahl aus, der über einen Umlenkspiegel 2 auf einen rotierenden Spiegel 4 des Scanners 3 fällt. Der vom Scannerspiegel 4 aufgefächerte Laserstrahl liegt in der Zeichenebene der Fig. 2. Diese Ebene wird in der Folge Abbildungsebene genannt. Die optische Achse des Lasers 1 schließt mit der Abbildungsebene 6 einen Winkel von vorzugsweise etwa 20° ein, wodurch es möglich wird, den Laser 1, den Scanner 3 samt Scannerspiegel 4, die Ablenkspiegel 7 und den Sensor 8 günstig in verschiedenen Ebenen anzuordnen. Der vom Laser 1 kommende Lichtstrahl verläuft vom Umlenkspiegel 2 an in der Abbildungsebene 6, der Spiegel 2 steht daher nicht normal auf diese Ebene.

Wenn, wie oben angeführt, die Ablenkspiegel 7 nicht normal zur Abbildungsebene 6 stehen, verlaufen die ausfallenden Strahlen zwischen diesen Spiegeln und dem Auge auch nicht in der Abbildungsebene. So kann ausgeglichen werden, daß der Schnitt der Abbildungsebene mit dem Auge kein exakter Radius bzw. Durchmesser ist. Diese schwache Schrägstellung ist in der Zeichnung nicht berücksichtigt.

Wie aus Fig. 2 ersichtlich ist, sind zwei der Ablenkspiegel nicht auf das Auge 9 gerichtet, sondern auf einen Kontrollsensor 10. Die beiden Kontrollspiegel 11, 12 lenken den durchgehenden Laserstrahl auf den Kontrollsensor 10, der den Strahldurchgang zeitgenau feststellt und so eine genaue Korrelation des auf das Auge 9 einfallenden Strahles mit dem vom Auge 9 auf den Sensor 2 reflektierten Strahl ermöglicht. Es ist selbstverständlich möglich, einen dritten Kontrollspiegel vorzusehen oder andere der Ablenkspiegel 7 als Kontrollspiegel zu justieren. Es können die Spiegel 11, 12 unabhängig von den Spiegeln 7 eine Schrägstellung aufweisen.

Es ist weiters möglich, den Scannerspiegel 4 näher an die Rotationsachse 5 heranzuführen und symmetrisch zu den Ablenkspiegeln 7 eine zweite Reihe von Ablenkspiegeln vorzusehen, um eine größere Anzahl von Meßpunkten pro Umdrehung des Scannerspiegels 4 abzutasten. Es ist auch möglich, bei dieser, aber auch bei der gezeigten Anordnung jeweils zwei oder auch mehr der Ablenkspiegel auf den gleichen Punkt der Hornhautoberfläche zu richten, um die Redundanz und damit die Meßgenauigkeit und Meßsicherheit weiter zu verbessern.

Es ist auch möglich, die Ablenkspiegel 7 so zu richten, daß der austretende Strahl die Achse 5 (Fig. 2) kreuzt und auf die (in Fig. 2) obere Augenhälfte fällt. Dadurch kann unter Umständen eine weitere Verbesserung des Strahleneitrittswinkels erzielt werden.

Es ist selbstverständlich auch eine Kombination der erwähnten Varianten möglich.

Die Auswertung der Meßergebnisse erfolgt auf durchaus übliche Art mit Hilfe eines Computers, der in Kenntnis der Geometrie der Meßanordnung, der Drehgeschwindigkeit des Spiegels 4 und der zeitlichen und räumlichen Meßergebnisse am Kontrollsensor 10 und am Sensor 8 die räumliche Lage des jeweiligen Meßpunktes am Auge 9 berechnet. Darüberhinaus kann der Computer selbstverständlich alle weiteren Berechnungen, die zur Bildung eines Hornhautmodelles notwendig sind, durchführen, beispielsweise die Tangentialebene in einzelnen Punkten oder die Krümmung der Hornhaut nach vorgegebenen Ebenen in vorgebenen Punkten bestimmen.

Wie aus Fig. 2 ersichtlich, ist es bei der erfindungsgemäßen Meßanordnung auch möglich, den direkt vom Scannerspiegel 4 erhaltenen Strahl direkt und ohne vorherige Reflexion an einem Ablenkspiegel 7 auf das Auge zu richten. Diese Möglichkeit gewinnt insbesondere dann an Bedeutung, wenn der Scannerspiegel 4 besonders nahe an der Rotationsachse 5 sitzt oder besonders großen Abstand von dieser Achse aufweist, da es jeweils dann möglich ist, eine günstige Reflexion für den Sensor 8 am Auge zu erhalten.

Der in Fig. 2 direkt auf der Achse 5 sitzende Ablenkspiegel 13 kann zur Ermittlung der Lage des vordersten Augenpunktes herangezogen werden.

Der Sensor 8 kann auf vielfache Weise aufgebaut sein, es ist auch möglich, zwei oder mehr lineare Sensoren 8 in unmittelbarer Nachbarschaft parallel zueinander, aber in axialer Richtung mit passend versetzten Meßabschnitten anzuordnen, um die Lage des vom Auge 9 reflektierten Strahles genau erfassen zu können. Dabei ist es selbstverständlich möglich, daß der Computer eine Interpolation bzw. Korrelation, der von den einzelnen Linearsensoren kommenden Signale vornimmt.

Da der Kontrollsensor 10 den von den Kontrollspiegeln 11, 12 reflektierten Strahl in einer genau vorherbestimmbaren Ebene empfängt, sind derartige Anordnungen bei ihm nicht notwendig. Wesentlich ist, daß der Sensor nach seiner Empfindlichkeit und Meßgeschwindigkeit zu gewählt wird, daß er den Zeitpunkt des Strahlendurchganges exakt feststellt.

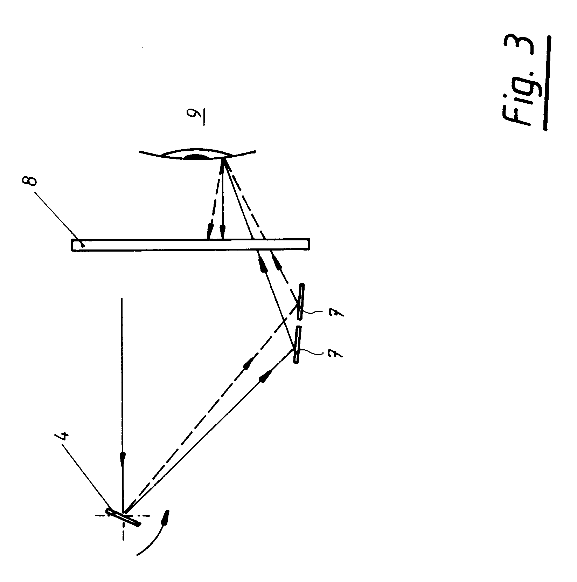

Wie aus den Abbildungen 1 und 3 hervorgeht, befindet sich der Sensor 8 nahe des Auges 9, sodaß der zu vermessende Strahl durch die doppelt positiv gewölbte Hornhaut der Oberfläche noch nicht so aufgeweitet ist, daß es die Meßgenauigkeit beeinflussen könnte.

Die Erfindung kann vom dargestellten Ausführungsbeispiel in vielerlei Hinsicht abweichend ausgeführt werden. So ist es möglich, einen anderen Winkel zwischen der Laserachse und der Abbildungsebene vorzugeben. Die Ablenkspiegel 7 sind, so wie die anderen Spiegel, handelsübliche Ware, die zur Reflexion von Laserlicht geeignet ist. Es ist sogar denkbar, statt des Lasers eine Lichtquelle zu verwenden, die einen scharf gebündelten Strahl abgibt, dessen Wellenlängenzusammensetzung so ist, daß keine unzulässige Aufweitung bei der Reflexion am Auge 9 erfolgt. Es wird aber die Verwendung eines Lasers bevorzugt.

Es ist auch möglich, auf den Umlenkspiegel 2 zu verzichten, doch muß dann der Laser und seine Strahlachse in der Abbildungsebene 6 liegen, was eine kompakte Anordnung erschwert. Andererseits ist es möglich, mehrere Umlenkspiegel vorzusehen, um bei der Laseranordnung freie Hand zu bekommen.

Das Verdrehen der Anordnung um die Rotationsachse 5 erfolgt vorzugsweise mittels eines Schrittmotors, der mit oder ohne zwischengeschaltetem Getriebe auf die Meßanordnung einwirkt. Die jeweilige Winkellage des Meßsystems bezüglich der Roationsachse 5 wird selbstverständlich auch an den Computer weitergeleitet.

Das beschriebene Meßsystem eignet sich nicht nur zur Vermessung der Hornhautoberfläche, sondern für alle kleinen Flächen bzw. Körper, die über reflektierende Flächen verfügen, insbesonders über gekrümmte Flächen.

## Patentansprüche

1. Optisches Meßsystem für die Bestimmung der Geometrie der Hornhaut eines Auges, mit einer Lichtquelle (1), einem Drehspiegel (4) und einer Ablenkoptik (7), die so angeordnet sind, daß ein von der Lichtquelle ausgehender Lichtstrahl nacheinander über den Drehspiegel und die Ablenkoptik auf die zu vermessende Hornhaut gerichtet wird, einem Sensor (8), der so angeordnet ist, daß an der Hornhaut reflektiertes Licht auf ihn auftrifft und mit einer mit dem Sensor verbundenen Auswerteeinheit zur Berechnung der Hornhautgeometrie aus den vom Sensor (8) übermittelten Meßsignalen, dadurch gekennzeichnet, daß die Ablenkoptik (7) ein System von festen ebenen Ablenkspiegeln (7) ist, bei dem die Flächen der einzelnen Spiegel (7) tangential an einer gekrümmten Linie angeordnet sind, welche in der vom rotierenden Lichtstrahl des Drehspiegels (4) erzeugten Ebene liegt, und daß mit zunehmender Entfernung eines Ablenkspiegels (7) von der Hornhaut der Lichtstrahl auf Punkte der Hornhautoberfläche gerichtet wird, die dem Scheitelpunkt der Hornhaut immer näher liegen.

2. Meßsystem nach Anspruch 1, dadurch gekennzeichnet, daß der Sensor (8) ein Linearsensor ist.

3. Meßsystem nach Anspruch 1 oder 2, dadurch gekennzeichnet, daß die Flächen der Ablenkspiegel (7) schiefwinkelig zur Ebene des rotierenden Lichtstrahles angeordnet sind und daß die Ablenkspiegel den Lichtstrahl exakt auf Punkte richten, welche einem bestimmten Radius bzw. Durchmesser einer Idealhornhautoberfläche entsprechen.

4. Meßsystem nach einem der Ansprüche 1 bis 3, dadurch gekennzeichnet, daß zumindest zwei der Ablenkspiegel (11, 12) den einfallenden Strahl auf ein Überwachungselement (10) für den Strahldurchgang lenken.

5. Meßsystem nach einem der Ansprüche 1 bis 3, dadurch gekennzeichnet, daß jeweils zwei, vorzugsweise benachbarte, Ablenkspiegel (7) auf ein- und denselben Punkt einer Idealhornhautoberfläche (9) gerichtet sind.

## Claims

1. Optical measuring system for the determination of the geometry of the cornea of an eye, with a light source (1), a rotating mirror (4) and a deflecting lens (7), which are arranged so that a light beam from the light source is directed one after the other via the rotating mirror and the deflecting lens onto the cornea to be measured, a sensor (8), which is arranged so that light reflected on the cornea strikes it and with an evaluation unit connected to the sensor for the calculation of the cornea geometry from the measuring signals transmitted by the sensor (8), characterised by the fact that the deflecting lens (7) is a system of fixed flat deflecting mirrors (7), where the surfaces of the individual mirrors(7) are arranged tangentially to a curved line, which is located in the plane produced by the rotating light beam of the rotating mirror (4), and by the fact that as a deflecting mirror (7) is increasingly moved away from the cornea, the light beam is directed onto points of the cornea surface, which are allocated increasingly nearer to the vertex of the cornea.

2. Measuring system in accordance with Claim 1, characterised by the fact that the sensor (8) is a linear sensor.

3. Measuring system in accordance with Claims 1 or 2, characterised by the fact that the surfaces of the deflecting mirror (7) are arranged at an oblique angle to the plane of the rotating light beam and that the deflecting mirror directs the light beam precisely to points, which correspond to a particular radius or diameter of an ideal cornea surface.

4. Measuring system in accordance with any of Claims 1 to 3 characterised by the fact that at least two of the deflecting mirrors (11, 12) deflect the incoming beam onto a monitoring element (10) for the beam passage.

5. Measuring system in accordance with any of Claims 1 to 3, characterised by the fact that in each case two, preferably neighbouring, deflecting mirrors (7) are directed to one and the same point of an ideal cornea surface (9)

## Revendications

1. Instrument de mesure optique pour la détermination de la géométrie d'une cornée oculaire avec une source lumineuse (1), un miroir tournant (4) et un déviateur optique (7) qui sont agencés de manière qu'un faisceau lumineux issu de la source soit dirigé successivement par le miroir tournant et le déviateur optique vers la cornée à mesurer, avec un capteur (8) qui est agencé de manière à recevoir la lumière réfléchie par la cornée et avec une unité de traitement connectée au capteur pour calculer la géométrie de la cornée à l'aide des signaux de mesure transmis par le capteur (8), instrument caractérisé en ce que le déviateur optique (7) est un système de miroirs déviateurs (7) plans fixes dont les plans particuliers sont disposés tangents à une ligne courbe qui est orientée dans le plan engendré par le faisceau lumineux tournant du miroir tournant (4) et en ce que le faisceau lumineux est dirigé, par un miroir déviateur (7) de distance croissante à la cornée, vers des points de la surface de la cornée qui se rapprochent toujours plus de son sommet.

2. Instrument de mesure selon la revendication 1 caractérisé en ce que le capteur (8) est un capteur linéaire.

3. Instrument selon la revendication 1 ou 2 caractérisé en ce que les plans des miroirs déviateurs (7) sont inclinés sur le plan du faisceau lumineux tournant et en ce que les miroirs déviateurs dirigent exactement le faisceau lumineux sur des points qui réprésentent un rayon ou un diamètre déterminé d'une surface de cornée théorique.

4. Instrument selon l'une des revendications 1 à 3 caractérisé en ce que au moins deux des miroirs déviateurs (11, 12) dévient le faisceau incident sur un élément de contrôle (10) de la trajectoire du faisceau.

5. Instrument selon l'une des revendications 1 à 3 caractérisé en ce que deux miroirs déviateurs (7) correspondants, de préférence voisins, sont dirigés vers un seul et unique point d'une surface de cornée théorique (9).
